(19) Europäisches Patentamt

European Patent Office

Office européen des brevets

(11) Veröffentlichungsnummer: **0 515 485 B1**

## (12) EUROPÄISCHE PATENTSCHRIFT

(45) Veröffentlichungstag der Patentschrift: **30.11.94**

(21) Anmeldenummer: **91904435.4**

(22) Anmeldetag: **13.02.91**

(86) Internationale Anmeldenummer:
**PCT/EP91/00277**

(87) Internationale Veröffentlichungsnummer:
**WO 91/13050 (05.09.91 91/21)**

(51) Int. Cl.⁵: **C07C 29/149**, C07C 31/125

### (54) VERFAHREN ZUM HYDRIEREN.

(30) Priorität: **22.02.90 DE 4005629**

(43) Veröffentlichungstag der Anmeldung:
**02.12.92 Patentblatt 92/49**

(45) Bekanntmachung des Hinweises auf die
Patenterteilung:
**30.11.94 Patentblatt 94/48**

(84) Benannte Vertragsstaaten:
**BE DE ES FR GB IT NL**

(56) Entgegenhaltungen:
**EP-A- 0 073 129**
**EP-A- 0 334 118**
**EP-A- 0 350 763**

(73) Patentinhaber: **Henkel Kommanditgesellschaft
auf Aktien**

**D-40191 Düsseldorf (DE)**

(72) Erfinder: **CARDUCK, Franz-Josef, Dr.
Landstrasse 18
W-5657 Haan (DE)**
Erfinder: **GÖBEL, Gerd, Dr.
Falkenweg 6
D-5000 Köln (DE)**
Erfinder: **FLECKENSTEIN, Theo, Dr.
Pfitzner Strasse 9
D-4010 Hilden (DE)**
Erfinder: **KREUTZER, Udo, Dr.
Robert-Koch-Strasse 1
D-4019 Monheim (DE)**
Erfinder: **DEMMERING, Günther, Dr.
Von-Galen-Strasse 40
D-5650 Solingen-Gräfrath (DE)**

**Beschreibung**

Die Erfindung betrifft ein Verfahren zum Hydrieren von nativen Fetten, Ölen und Fettderivaten wie Fettsäuren und Fettsäureestern zu Fettalkoholen im Festbettreaktor, insbesondere im Gleichstrom, wobei Wasserstoff im stöchiometrischen Überschuß, insbesondere mit 10 bis 100fachem Überschuß, im Kreis gefahren wird und das Fett, Öl oder Fettderivat den Reaktor nur einmal durchläuft.

Bei einem derartigen bekannten Verfahren, z. B. zum Hydrieren von Fettsäuremethylester, findet die eigentliche Hydrierreaktion in einem oder mehreren hintereinander geschalteten Festbettreaktoren bei Temperaturen von 200 bis 250 °C und einem Wasserstoffdruck von 200 bis 300 bar statt. Dazu wird der Fettsäuremethylester mit Preßpumpen in die Anlage gedrückt, wo er mit komprimiertem Wasserstoff vermischt, gemeinsam mit diesem auf Reaktionstemperatur erhitzt und von oben in den Reaktor geleitet wird.

Nach Durchlaufen der Reaktoren wird das Reaktionsgemisch gekühlt und in einem Abscheider in die Flüssigphase und die Gasphase aufgetrennt. Die Flüssigphase wird entspannt und zur Methanolabtrennung geleitet, während die hauptsächlich aus Wasserstoff bestehenden Gasphase über einen Kompressor im Kreis geführt wird. In der als Verdampferanlage ausgelegten Methanolabtrennung wird der Fettalkohol vom Methanol befreit und kann dann ohne weitere Reinigung seiner Verwendung zugeführt werden (vgl. **H.Komp und H.P.Kubersky in: "Fettalkohole", Henkel KGaA (ed.), Eigenverlag, 1981, S.51 bis 79).**

Jedoch ist bei der katalytischen Hydrierung von Fetten, Ölen und Fettderivaten zu Fettalkoholen im Festbettreaktor (Tricklebed-Fahrweise) insbesondere bei adiabatischer Reaktionsführung ein hoher, 10 bis 100fach über dem stöchiometrischen Bedarf liegender Wasserstoffüberschuß erforderlich, damit durch den hohen Gasüberschuß ein Großteil der Reaktionswärme abgeführt wird. Übertemperaturen im Katalysatorbett führen nämlich vermehrt zu Nebenprodukten und verkürzen die Standzeit des Katalysators.

In technischen Anlagen wird der Wasserstoff aus wirtschaftlichen Gründen rezirkuliert. Hierzu muß der Wasserstoff nach Passieren des Reaktors von 230 °C auf ca. 50 °C abgekühlt, mit dem Kreisgaskompressor verdichtet und im Spitzenerhitzer erneut auf Reaktionstemperatur aufgeheizt werden. Die Wirtschaftlichkeit eines solchen Hydrierverfahrens in Bezug auf Investions- und Betriebskosten wird daher wesentlich durch den spezifischen Kreisgasbedarf ($Nm^3$ $H_2$/t Reaktionsprodukt) bestimmt.

Der Erfindung liegt daher die Aufgabe zugrunde, durch verfahrenstechnische Maßnahmen den spezifischen Wasserstoffbedarf zu minimieren, ohne hierbei wesentliche Einbußen in Bezug auf spezifische Reaktorbelastung, Produktselektivität und Katalysatorstandzeit hinnehmen zu müssen.

Diese Aufgabe wird erfindungsgemäß bei einem Verfahren der Eingangs genannten Art dadurch gelöst, daß der Wasserstoff durch mindestens zwei Festbettreaktoren hintereinander geführt wird, ohne daß das aus den Reaktoren austretende und in die nachfolgenden Reaktoren eintretende Gas abgekühlt wird, und daß das Fett, Öl oder Fettderivat parallel in die Reaktoren eingespeist wird.

Das erfindungsgemäße Verfahren kann unabhängig von der speziellen Ausführungsform des Reaktors, z. B. als Schachtreaktor oder Rohrbündelreaktor, sowie unabhängig von dessen Betriebsweise, z. B. adiabatisch oder isotherm betrieben werden. Bei Hydrieranlagen mit n Reaktoren wird die spezifische Kreisgasmenge auf 1/n verringert. Die Anreicherung verdampfbarer Anteile in der Gasphase führt nur zu einer geringen Leistungseinbuße von etwa 5 bis 10 % für den zweiten Reaktor und die nachfolgenden Reaktoren. Die Selektivität des Katalysators sowie dessen Standzeitverhalten werden nicht signifikant beeinflußt.

Ferner wird vorgeschlagen, daß das Verfahren mit zwei Reaktoren betrieben wird.

In einer weiteren vorteilhaften Ausführungsform des erfindungsgemäßen Verfahrens werden von dem aus den vorhergehenden Reaktoren austretenden Gas die flüssigen Reaktionsprodukte abgeschieden, bevor das Gas in den jeweils nachfolgenden Reaktor eintritt. Dabei ist es besonders vorteilhaft, wenn die von dem Gas abgeschiedenen flüssigen Reaktionsprodukte dem aus dem letzten Reaktor austretenden Gas zugeführt, zusammen mit diesem abgekühlt, in eine flüssige Phase und eine gasförmige Phase aufgetrennt werden und die letztere dann in den ersten Reaktor zurückgeführt wird.

Die Erfindung betrifft zum anderen eine Anlage zum Hydrieren von aus nativen Fetten, Ölen und Fettderivaten wie Fettsäure und Fettsäureester bestehenden Einsatzprodukten zu Fettalkoholen, mit mindestens einem Festbettreaktor zum Hindurchleiten des Einsatzprodukts sowie des Wasserstoffs, mit dem Reaktor vorgeschalteten Erhitzern für das Einsatzprodukt und für den Wasserstoff, mit einer Kühleinrichtung für den Reaktorablauf und einem nachgeschalteten Abscheider zur Gas-Flüssigkeitstrennung, der zum Rückführen des Gases an den Reaktor angeschlossen ist.

Die erfindungsgemäße Aufgabe wird bei dieser Anlage durch mindestens zwei hintereinandergeschaltete Festbettreaktoren, die über jeweils einen Abscheider miteinander verbunden sind, dessen Flüssigkeitsablauf zum Ablauf des letzten Reaktors geführt ist, und durch eine parallele Zuführung des Einsatzproduktes zu jedem der Reaktoren gelöst.

Vorteilhaft sind zwei hintereinander geschaltete Festbettreaktoren.

Insbesondere wird vorgeschlagen, daß jede Zuführung des Einsatzproduktes eine Pumpe und einen Wärmeaustauscher aufweist. Dadurch sind die Feedmenge und Feedtemperatur für jeden Reaktor auf einfache Weise getrennt einstellbar.

Ausführungsbeispiele der Erfindung sind nachfolgend anhand der Figuren näher erläutert. Es zeigen

Figur 1 die Katalysatorbelastung in einem Diagramm durch Angabe des Temperaturverlaufs über dem Verhältnis Feed-Volumen/Katalysator-Volumen,

Figur 2 die Katalysatorbelastung, ausgedrückt in LHSV und aufgetragen über die Betriebstage bei der Direkthydrierung von Kokosöl,

Figur 3 den Temperaturverlauf und die Ausbeute an 1,2-Propandiol bei der Kokosöldirekthydrierung, aufgetragen über die Betriebstage und

Figur 4 die Verseifungszahl bei dieser Direkthydrierung, aufgetragen ebenfalls über die Betriebstage und

Figur 5 ein Beispiel für die erfindungsgemäße Anlage, schematisch dargestellt.

In einer 2 x 20-Liter-Doppelreaktoranlage wurden Ausführungsbeispiele des erfindungsgemäßen Verfahrens gefahren. Dabei wurde Kokosöl direkt hydriert, wobei Fettalkohole und 1,2-Propandiol entstehen.

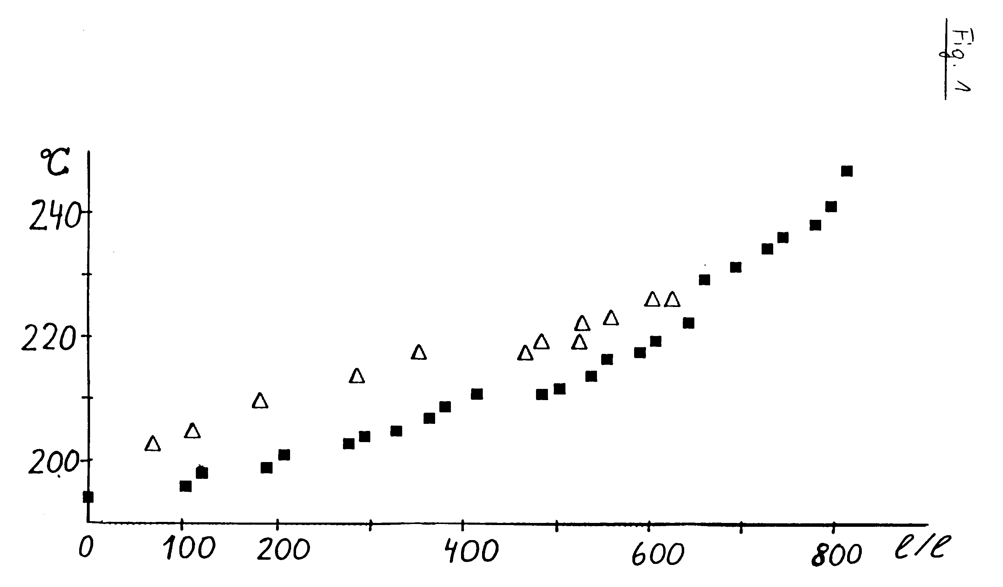

In Figur 1 sind die Ergebnisse hinsichtlich der Katalysator-Lebensdauer beim erfindungsgemäßen Verfahren, das mit LHSV = 0,71 betrieben wurde, durch Dreiecke dargestellt. Ein Vergleichsbeispiel in üblicher Fahrweise mit LHSV = 0,72 ist durch die Quadrate gekennzeichnet. Es ist ersichtlich, daß die Katalysator-Lebensdauer durch das erfindungsgemäße Verfahren nicht wesentlich verkürzt wird.

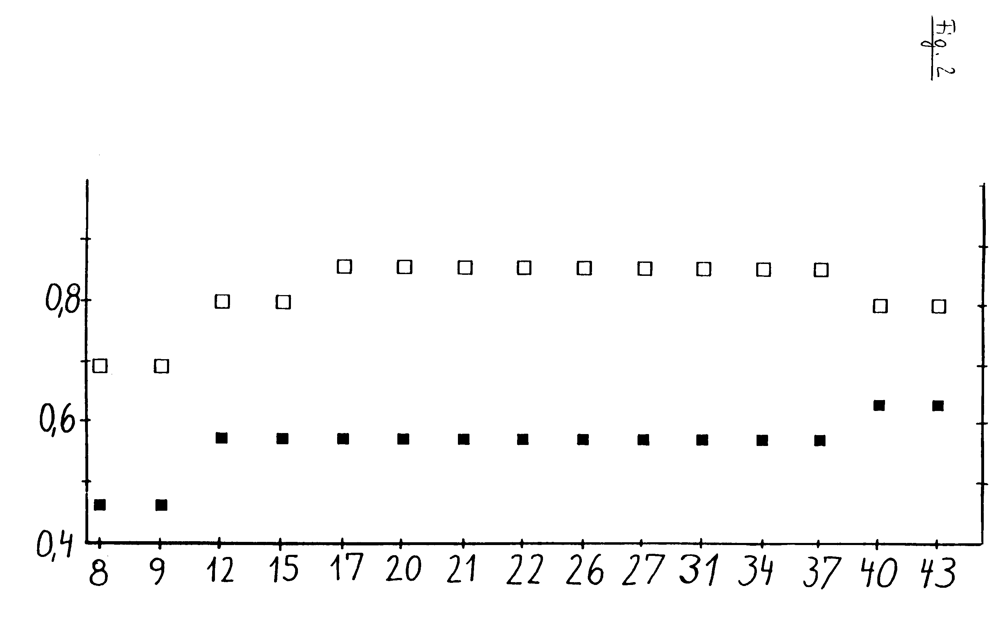

In Figur 2 ist die Katalysatorbelastung für den ersten Reaktor durch offene Quadrate und für den nachfolgenden Reaktor, den zweiten Reaktor, durch ausgefüllte Quadrate dargestellt. Diese LHSV-Werte zeigen, daß bei dem nachfolgenden Reaktor noch hohe Katalysatorbelastungen erreichbar sind.

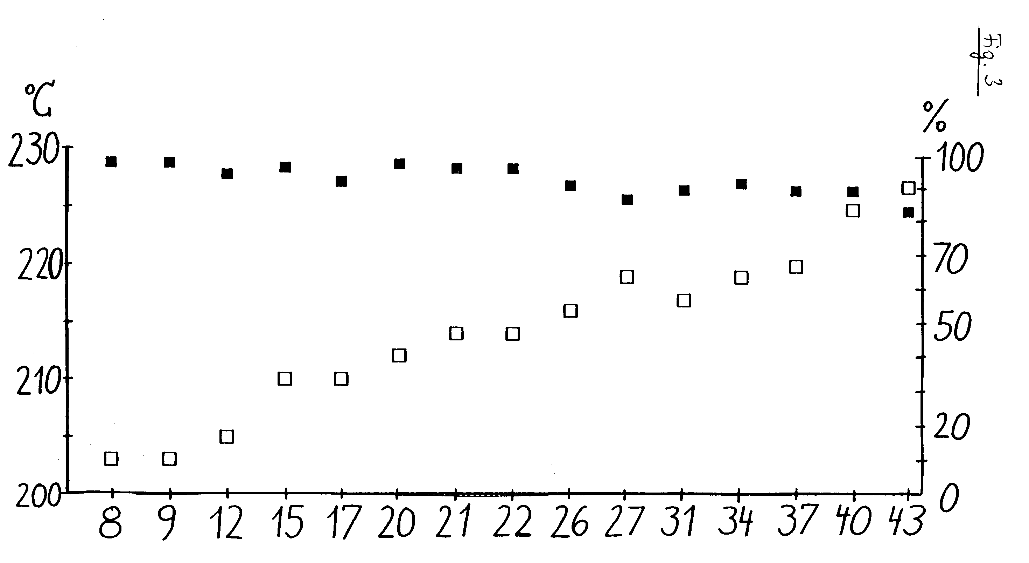

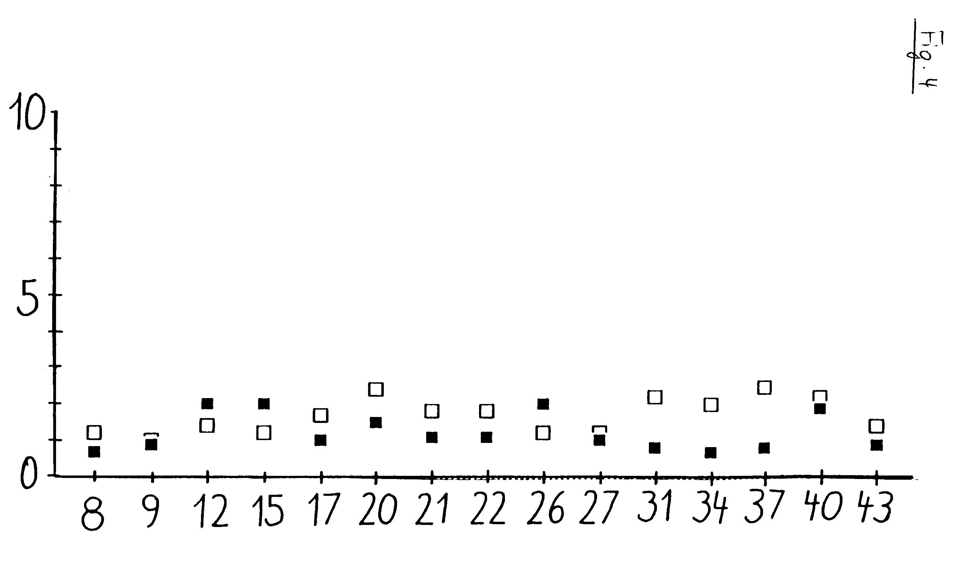

In Figur 3 ist die Temperatur durch die offenen Quadrate und die Ausbeute an 1,2 Propandiol durch die ausgefüllten Quadrate dargestellt. Auch nach längerer Betriebsdauer erhält man noch eine gute Ausbeute. Dies wird auch in Figur 4 deutlich, in der die Verseifungszahl für den ersten Reaktor durch die offenen Quadrate und den zweiten Reaktor durch die ausgefüllten Quadrate dargestellt ist.

Nähere Daten zum Ausführungsbeispiel finden sich in Tabelle 1.

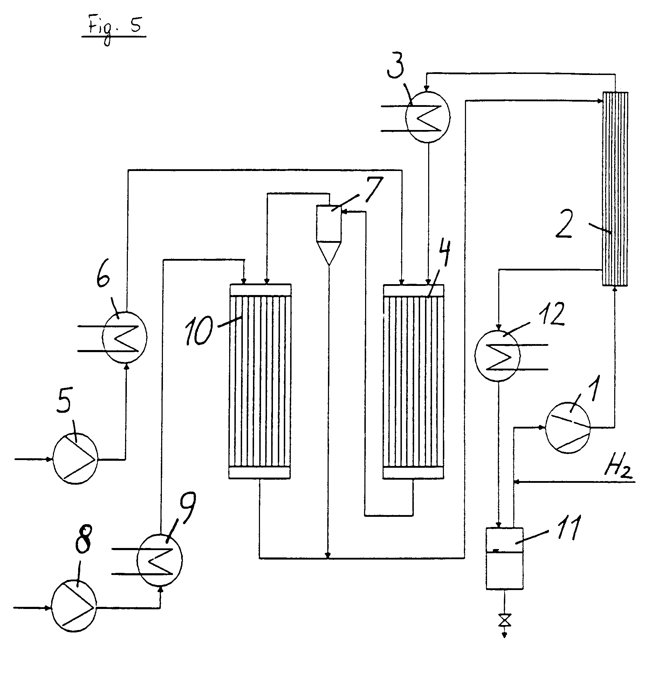

Figur 5 zeigt die Schaltung der Reaktoren in einer schematischen Darstellung. Von einer Gasumlaufpumpe 1 wird frischer Wasserstoff zusammen mit Kreis-Wasserstoff über den Gegenstromwärmeaustauscher 2 und den Spitzenerhitzer 3 in den ersten Reaktor 4 eingespeist. In diesem Reaktor wird gleichzeitig der Einsatzstoff von der Pumpe 5 über den Erhitzer 6 gedrückt. Das heiße Reaktionsgas wird durch den Zentrifugalabscheider 7 vom flüssigen Reaktionsprodukt befreit und zusammen mit dem Einsatzstoff über die Pumpe 8 und den Erhitzer 9 durch den zweiten Reaktor 10 geleitet. Flüssige und gasförmige Reaktionsprodukte werden über den Gegenstromwärmeaustauscher 2 und den Kühler 12 in den Hochdruckabscheider 11 geführt. Das Reaktionsgas wird zusammen mit dem Frischwasserstoff im Kompressor 1 verdichtet und im Kreis gefahren.

# T a b e l l e   1

Katalysator-Typ     : CuCr

Kata-Form           : Extrudat 3 mm

| Reaktor | 1 | 2 | 1 + 2 |
|---|---|---|---|
| Bimssteine unten | 500 ml | 500 ml | 1000 ml |
| Kata Menge | 21,04 kg | 20,94 kg | 41,98 kg |
| Kata Volumen | 17,53 kg | 17,44 l | 35 l |
| Füllvorgang | Vibrations-rinne | Vibrations-rinne | Vibrations-rinne |
| Füllzeit | 5 min. | 5 min. | |
| Staubanteil | 2,40 % | 0,98 % | 1,68 % |
| Bimssteine oben | 100 ml | 100 ml | 200 ml |
| Leervolumen | 200 ml | 200 ml | 400 ml |

Nachfolgend werden in tabellarischer Form die Ausgangsdaten und Ergebnisse einer Hydrierung von Fettsäuremethylester gemäß der Erfindung angegeben.

Angaben zum Einsatzstoff

$C_{12}$-$C_{18}$-Fettsäuremethylester (dest.) aus Palmkernöl

| Kennzahlen | Verseifungszahl | 244 |
|---|---|---|
| | Jodzahl | 16 |

Reaktordaten

| Reaktorvolumen | 2 x 20 1 Doppelrohranlage |
|---|---|
| Länge | 2 x 6 m |

Reaktionsdaten

| Reaktionstemperatur | 185 °C |
|---|---|
| Reaktionsdruck | 250 bar |
| LHSV | 0,5 ($h^{-1}$) |
| Wasserstoff-Kreisgas-Menge | 2,0 cbm/h (bei 250 bar/60 °C) |

Reaktionsproduktdaten

$C_{12}$-$C_{18}$-Fettalkohol/Methanol-Gemisch

Analyse-Kennzahlen (nach Methanolabtrieb)

| Verseifungszahl | - Reaktor 1 | 0,2 |
|---|---|---|
| | - Reaktor 2 | 0,3 |

Die Jodzahlen beider Proben lagen unter 1; der Kohlenwasserstoffgehalt lag unter 0,2 Gew.-%.

Bezugszeichenliste

1    Gasumlaufpumpe
2    Gegenstromwärmeaustauscher
3    Spitzenerhitzer
4    erster Reaktor
5    Pumpe
6    Erhitzer
7    Zentrifugalabscheider
8    Pumpe
9    Erhitzer
10    zweiter Reaktor
11    Hochdruckabscheider
12    Kühler

**Patentansprüche**

**1.** Verfahren zum Hydrieren von nativen Fetten, Ölen und Fettderivaten wie Fettsäuren und Fettsäureestern zu Fettalkoholen im Festbettreaktor, insbesondere im Gleichstrom, wobei Wasserstoff im stöchiometrischen Überschuß, insbesondere mit 10 bis 100fachem Überschuß, im Kreis gefahren wird und das Fett, Öl oder Fettderivat den Reaktor nur einmal durchläuft,
dadurch gekennzeichnet,
daß der Wasserstoff durch mindestens zwei Festbettreaktoren (4, 10) hintereinander geführt wird, ohne daß das aus den Reaktoren (4) austretende und in die nachfolgenden Reaktoren (10) eintretende Gas abgekühlt wird, und daß das Fett, Öl oder Fettderivat parallel in die Reaktoren (4, 10) eingespeist wird.

2. Verfahren nach Anspruch 1,
dadurch gekennzeichnet,
daß das Verfahren mit zwei Reaktoren (4, 10) betrieben wird.

3. Verfahren nach Anspruch 1,
dadurch gekennzeichnet,
daß von dem aus den vorhergehenden Reaktoren (4) austretenden Gas die flüssigen Reaktionsprodukte abgeschieden werden, bevor das Gas in den jeweils nachfolgenden Reaktor (10) eintritt.

4. Verfahren nach Anspruch 3,
dadurch gekennzeichnet,
daß die von dem Gas abgeschiedenen flüssigen Reaktionsprodukte dem aus dem letzten Reaktor (10) austretenden Gas zugeführt, zusammen mit diesem abgekühlt, in eine flüssige Phase und eine gasförmige Phase aufgetrennt werden und die letztere dann in den ersten Reaktor (4) zurückgeführt wird.

5. Anlage zum Hydrieren von aus nativen Fetten, Ölen und Fettderivaten wie Fettsäure und Fettsäureester bestehenden Ein satzprodukten zu Fettalkoholen,
mit mindestens einem Festbettreaktor (4, 10) zum Hindurchleiten des Einsatzprodukts sowie des Wasserstoffs, mit dem Reaktor (4, 10) vorgeschalteten Erhitzern (2, 3, 6, 9) für das Einsatz produkt und für den Wasserstoff,
mit einer Kühleinrichtung (2, 12) für den Reaktorablauf und einem nachgeschalteten Abscheider (11) zur Gas-Flüssigkeitstrennung, der zum Rückführen des Gases an den Reaktor angeschlossen ist, gekennzeichnet durch
mindestens zwei hintereinandergeschaltete Festbettreaktoren (4, 10) die über jeweils einen Abscheider (7) miteinander verbunden sind, dessen Flüssigkeitsablauf zum Ablauf des letzten Reaktors (10) geführt ist,
und durch eine parallele Zuführung des Einsatzprodukts zu jedem der Reaktoren (4, 10).

6. Anlage nach Anspruch 5,
gekennzeichnet durch
zwei hintereinandergeschaltete Festbettreaktoren (4, 10).

7. Anlage nach Anspruch 5,
dadurch gekennzeichnet,
daß jede Zuführung des Einsatzproduktes eine Pumpe (5, 8) und einen Wärmeaustauscher (6, 9) aufweist.

**Claims**

1. A process for the hydrogenation of native fats, oils and fat derivatives, such as fatty acids and fatty acid esters, to fatty alcohols in a fixed-bed reactor, more particularly in co-current, hydrogen being recirculated in a stoichiometric excess, more particularly in an excess of 10 to 100 fold, and the fat, oil or fat derivative passing through the reactor only once, characterized in that the hydrogen is passed successively through at least two fixed-bed reactors (4,10) without the gas issuing from the reactors (4) and entering the following reactors (10) being cooled and in that the fat, oil or fat derivative is simultaneously introduced into the reactors (4,10).

2. A process as claimed in claim 1, characterized in that the process is carried out with two reactors (4,10).

3. A process as claimed in claim 1, characterized in that the liquid reaction products are separated from the gas issuing from the preceding reactors (4) before the gas enters the following reactor (10).

4. A process as claimed in claim 3, characterized in that the liquid reaction products separated from the gas are introduced into the gas issuing from the reactor (10), cooled with that gas, separated into a liquid phase and a gas phase and the gas phase is subsequently returned to the first reactor (4).

**5.** A plant for the hydrogenation of starting products consisting of fats, oils and fat derivatives, such as fatty acid and fatty acid ester, to fatty alcohols, comprising at least one fixed-bed reactor (4,10) through which the starting product and the hydrogen are passed, heaters (2,3,6,9) preceding the reactor (4,10) for the starting product and the hydrogen, a cooling system (2,12) for the reactor outflow and a following separator (11) for gas/liquid separation which is connected to the reactor for recirculating the gas, characterized by at least two fixed-bed reactors (4,10) arranged in tandem which are connected to one another by a separator (7) of which the liquid outflow is conducted to the outflow of the last reactor (10) and by a parallel feed line for delivering the starting product to each of the reactors (4,10).

**6.** A plant as claimed in claim 5, characterized by two fixed-bed reactors (4,10) arranged in tandem.

**7.** A plant as claimed in claim 5, characterized in that each feed line for the starting product comprises a pump (5,8) and a heat exchanger (6,9).

**Revendications**

**1.** Procédé d'hydrogénation de graisses naturelles, d'huiles et de dérivés gras comme des acides gras et des esters d'acides gras, en alcools gras dans un réacteur en lit fixe, notamment en écoulement de même sens, l'hydrogène est introduit en excès stoechiométrique dans le circuit, notamment avec un excès de 10 à 100 fois, et la graisse, l'huile ou le dérivé gras ne traverse qu'une fois le réacteur, caractérisé en ce qu'on fait passer l'hydrogène successivement dans au moins deux réacteurs à lit fixe (4, 10), sans refroidir le gaz qui sort des réacteurs (4) et qui entre dans les réacteurs suivants (10) et qu'on introduit la graisse, l'huile ou le dérivé gras en parallèle dans les réacteurs (4, 10).

**2.** Procédé selon la revendication 1, caractérisé en ce qu'on fait fonctionner le procédé avec deux réacteurs (4, 10).

**3.** Procédé selon la revendication 1, caractérisé en ce qu'on sépare les produits de réaction liquides du gaz sortant des réacteurs en amont (4), avant que le gaz ne pénètre dans le réacteur suivant (10).

**4.** Procédé selon la revendication 3, caractérisé en ce qu'on introduit les produits de réaction liquides séparés du gaz dans le gaz sortant du dernier réacteur (10), on les refroidit ensemble, on les fractionne en une phase liquide et une phase gazeuse et on recycle cette dernière dans le premier réacteur (4).

**5.** Unité d'hydrogénation de graisses naturelles d'huiles et de dérivés gras comme des acides gras et des esters d'acide gras qui constituent les produits de départ, en alcools gras, avec au moins un réacteur en lit fixe (4, 10) pour y faire passer le produit de départ, ainsi que l'hydrogène, avec des réchauffeurs (2, 3, 6, 9) montés en amont du réacteur (4, 10) pour le produit de départ et pour l'hydrogène, avec un dispositif de refroidissement (2, 12) pour le soutirage du réacteur et un séparateur (11) branché en aval pour séparer le gaz du liquide et qui est branché au réacteur pour recycler le gaz, caractérisé en ce qu'au moins deux réacteurs en lit fixe (4, 10) sont branchés successivement, et qui sont reliés par l'intermédiaire d'un séparateur (7), dont le soutirage de liquide est relié au soutirage du dernier réacteur (10) et qu'il existe une introduction parallèle du produit de départ dans chaque réacteur (4, 10).

**6.** Unité selon la revendication 5, caractérisée par deux réacteurs en lit fixe (4, 10) branchés successivement.

**7.** Unité selon la revendication 5, caractérisée en ce que chaque introduction de produit de départ présente une pompe (5, 8) et un échangeur de chaleur (6, 9).

Fig. 1

EP 0 515 485 B1

Fig. 2

Fig. 3

Fig. 4

Fig. 5